(19) Europäisches Patentamt
European Patent Office
Office européen des brevets

(11) Veröffentlichungsnummer: **0 005 849**
**B2**

# (12) NEUE EUROPÄISCHE PATENTSCHRIFT

(45) Veröffentlichungstag der neuen Patentschrift: **16.07.86**

(51) Int. Cl.[4]: **C 07 C 102/00,** C 07 C 103/34

(21) Anmeldenummer: **79101725.4**

(22) Anmeldetag: **01.06.79**

(54) **Verfahren zur Herstellung von Alpha-Chlor-N-monoalkyl-acetoacetamiden.**

(30) Priorität: **01.06.78 DE 2824046**

(43) Veröffentlichungstag der Anmeldung: **12.12.79 Patentblatt 79/25**

(45) Bekanntmachung des Hinweises auf die Patenterteilung: **21.10.81 Patentblatt 81/42**

(45) Bekanntmachung des Hinweises auf die Entscheidung über den Einspruch: **16.07.86 Patentblatt 86/29**

(84) Benannte Vertragsstaaten: **CH DE FR GB IT NL**

(56) Entgegenhaltungen:
**DE - A - 1 493 022**
**DE - A - 1 618 207**
**DE - A - 2 049 045**
**GB - A - 2 000 499**
**US - A - 3 917 694**

**Die Akte enthält technische Angaben, die nach dem Eingang der Anmeldung eingereicht wurden und die nicht in dieser Patentschrift enthalten sind.**

(73) Patentinhaber: **WACKER-CHEMIE GMBH, Prinzregentenstrasse 22, D-8000 München 22 (DE)**

(72) Erfinder: **Künstle, Gerhard, Dr., Dipl.-Chem, Waldstrasse 29, D-8261 Raitenhaslach (DE)**
Erfinder: **Jung, Herbert, Bachstrasse 12, D-8263 Burghausen (DE)**

EP 0 005 849 B2

**Beschreibung**

α-Chlor-N-monoalkyl-acetoacetamide sind wertvolle Zwischenprodukte für die Herstellung von Insektiziden auf Grundlage von Phosphorsäureestern, insbesondere von Dialkylphosphaten der N-Monoalkyl-3-hydroxycrotonsäureamide (vgl. U.S.-PS 2 802 855).

Die Herstellung der α-Chloracetoacetamide erfolgte bisher durch Chlorierung der entsprechenden Acetoacetamide, wobei als Nebenprodukte jeweils beträchtliche Mengen der α,α-Dichloracetoacetamide gebildet wurden, deren Abtrennung aber aufgrund ähnlicher physikalischer Eigenschaften praktisch nicht möglich ist. Es sind daher bereits mehrere Verfahren bekannt geworden, die durch eine anschliessende selektive Dehalogenierung der α,α-Dichloracetoacetamide zu praktisch reinen α-Chloracetoacetamiden führen. Derartige Verfahren erfordern jedoch zusätzliche Massnahmen und sind daher mit einem unwirtschaftlich hohen Aufwand verbunden.

Nachdem in der DE-PS 1 493 022 (die der U.S.-PS 3 449 421 entspricht) beschriebenen Verfahren, das auf die Herstellung von α-Chlor-N,N-dialkylacetoacetamiden eingeschränkt ist, kann die Chlorierung der N,N-Dialkylacetoacetamide mit unterchloriger Säure, N-Chlorharnstoff und/oder tert. Butylhypochlorit bei Temperaturen von 0°C bis 80°C durchgeführt werden. Hierbei wird Wasser als geeignetes Lösungsmittel empfohlen, insbesondere dann, wenn als Chlorierungsmittel unterchlorige Säure, die in situ durch Reaktion von Chlor mit Wasser hergestellt werden kann, verwendet wird. Da die Reaktion im unteren Temperaturbereich zu langsam verläuft, wird das Verfahren vorteilhaft bei Raumtemperatur und darüber durchgeführt, wobei durch Mitverwendung von Harnstoff die Selektivität verbessert wird. Wie aus Beispiel 2 ersichtlich, werden unter diesen Bedingungen 96,4% α-Chlor-N,N-dimethylacetoacetamid neben 0,6% α,α-Dichlor-N,N-dimethylacetoacetamid und 1,1% N-N-Dimethylacetoacetamid (unumgesetztes Ausgangsmaterial) erhalten, was einer Umwandlung von 92,6% entspricht.

Die Übertragung dieses Verfahrens auf die Herstellung von α-Chlor-N-monalkylacetoacetamiden, wie α-Chlor-N-methyl-acetoacetamid war indessen nicht möglich ohne die Bildung von grossen Mengen des entsprechenden α,α-Dichlor-N-methyl-acetoacetamids. Aus der DE-PS 1 618 207 (die der U.S.-PS 3 483 252 entspricht) ist daher ein Verfahren bekannt geworden, nach dem N-Monoalkylacetoacetamide bei Temperaturen unter 0°C, das heisst, im Bereich von −8°C bis −25°C in Gegenwart von Wasser und Harnstoff chloriert werden und wobei zusätzlich die Gegenwart eines Alkohols erforderlich ist. Wie aus Beispiel 1 ersichtlich, werden unter diesen Bedingungen 90% α-Chlor-N-methylacetoacetamid neben 5% α,α-Dichlor-N-methylacetoacetamid und 1,6% unumgesetztes Ausgangsmaterial erhalten.

Aber auch dieses Verfahren konnte bei Übertragung in den grosstechnischen Bereich nicht befriedigen, da die Menge der als unerwünschte Nebenprodukte gebildeten α,α-Dichlorverbindungen noch immer zu gross ist und die benötigten grossen Harnstoffmengen (0,5 Mol je Mol eingesetztes Chlor) in hohem Masse unwirtschaftlich sind.

Nach dem in der DE-OS 2 049 045 (die der NL-Anmeldung 7 014 636 entspricht) beschirebenen Verfahren soll es hingegen möglich sein, sowohl N-Monoalkyl- als auch N,N-Dialkylacetoacetamide bei Temperaturen unter 0°C ohne Harnstoffzusatz zu chlorieren, wenn als Lösungsmittel ein Alkanol mit 1 bis 6 C-Atomen verwendet wird, das gegebenenfalls mit 5 bis 40% Wasser, vorzugsweise etwa 15 bis 20% Wasser enthält. Die Selektivität dieses Verfahrens in bezug auf die Bildung der gewünschten α-Monochlorderivate soll gegenüber den bisher bekannten Verfahren erhöht sein, da nur etwa 5% der α,α-Dichlorderivate gebildet werden, die gegebenenfalls nach Entfernen des Alkanols in bekannter Weise durch Behandeln mit einem Reduktionsmittel selektiv dehalogeniert werden können. Wie aus den Beispielen hervorgeht, die sich ausschliesslich mit der Chlorierung von N-Monomethylacetoacetamid befassen, wurden die besten Ergebnisse hinsichtlich Umsatz und Selektivität mit Äthanol als Lösungsmittel erreicht, das 7,4% Wasser enthielt. Eine Erhöhung des Wassergehaltes auf 50% oder gar 75% hatte indessen unter sonst nahezu gleichen Bedingungen ein deutliches Absinken des Umsatzes und der Selektivität zur Folge. Diese DE-OS vermittelt daher die Lehre, dass die Chlorierung von Acetoacetamiden, insbesondere von N-Monomethylacetoacetamid nur in Verdünnung mit einem Alkanol, dessen Wassergehalt 20% nicht übersteigen darf, bei Temperaturen unterhalb von −15°C unter Bildung der entsprechenden α-Monochlorderivate mit einem Umsatz von über 95% und einer Selektivität in der gleichen Grössenordnung in annehmbaren Reaktionszeiten, dass heisst, innerhalb von etwa 2 Stunden durchgeführt werden kann. Jede Veränderung dieser Reaktionsbedingungen, insbesondere die Erhöhung des Wassergehalts im vorgegebenen Lösungsmittel ist mit einer Verminderung des Umsatzes und/oder der Selektivität verbunden, wobei ausdrücklich darauf hingewiesen wird, dass mit steigendem Wassergehalt auch die Reaktionszeit zunimmt. Der Anwendung dieses Verfahrens sind daher in der Praxis enge Grenzen gesetzt. Ausserdem sind zusätzlich Massnahmen zur destillativen Entfernung des Alkanols erforderlich, bevor die Aufarbeitung des Reaktionsproduktes in bekannter Weise vorgenommen werden kann.

Diese engen Grenzen wurden offensichtlich von der Anmelderin selbst erkannt, wie aus den Ausführungen zum Stand der Technik in der U.S.-PS 3 917 694 hervorgeht, worin darauf hingewiesen wird, dass bei diesem Verfahren Umstz und Selektivität in vielen Fällen die Neigung haben abzunehmen, wenn die Reaktion in vergrössertem Massstab durchgeführt wird. In dieser

US-PS wird daher das Verfahren zur Chlorierung von N-Alkyl- oder N,N-Dialkylacetoacetamiden bei Temperaturen unter 0°C und einem Alkanol als Lösungsmittel dadurch abgewandelt, dass die Chloreinleitungsgeschwindigkeit bei Beginn begrenzt und mit fortschreitender Reaktion zunehmend gesteigert wird. Hierdurch soll eine gleichbleibende hohe Umwandlung und Selektivität erzielt werden. Wie aus den Beispielen ersichtlich, werden jedoch im Falle der Chlorierung von N-Monomethylacetoacetamid gute Ergebnisse nur dann erreicht, wenn gleichzeitig Harnstoff und zwar in Mengen von bis zu 1 Mol je Mol eingesetztes N-Monomethylacetoacetamid mitverwendet werden, dessen Bezeichnung als «Chlorierungskatalysator» aufgrund dieser grossen Menge nicht mehr gerechtfertigt ist.

Diese bekannten Verfahren beweisen, dass bei der Chlorierung von Acetoacetamiden mit Chlor die Selektivität in bezug auf die gewünschte Bildung der entsprechenden Monochlorverbindungen durch Senken der Reaktionstemperatur, Auswahl des Lösungsmittels und/oder Verlangsamung der Chloreinleitungsgeschwindigkeit positiv beeinflusst werden kann. Die Erzielung einer hohen Selektivität ist indessen in der Praxis nur dann sinnvoll, wenn sie nicht auf Kosten einer unvollständigen Umsetzung erkauft werden muss. Hohe Selektivität bei gleichzeitig hohem Umsatz können aber nach den bisher bekannten Verfahren, insbesondere bei der Chlorierung von N-Monoalkylacetoacetamiden, die hinsichtlich der unerwünschten Bildung von alpha,alpha-Dichlorverbindungen ungleich empfindlicher reagieren, als die entsprechenden N,N-Dialkylacetoacetamide, nur durch aufwendige Massnahmen erreicht werden.

Der Erfindung liegt daher die Aufgabe zugrunde, ein vereinfachtes Verfahren zur Herstellung von alpha-Chlor-N-monmoalkylacetoacetamiden der allgemeinen Formel

$CH_3COCH(Cl)CONR_1H$

worin $R_1$ Alkylrest mit 1 bis 3 C-Atomen bedeutet, durch Umsetzung von N-Monoalkyl-acetoacetamiden der allgemeinen Formel

$CH_3COCH_2CONR_1H$

worin $R_1$ die angegebene Bedeutung hat, mit Chlor in Gegenwart von Wasser enthaltenden Lösungsmitteln bei Temperaturen unter 0°C zur Verfügung zu stellen, das bei einem Umsatz von mindestens 95% eine hohe Selektivität von mindestens 97% gewährleistet, ohne dass hierzu aufwendige Massnahmen, wie die Einschaltung einer zusätzlichen Destillationsstufe zur Entfernung von Lösungsmittel, der Regulierung der Chloreinleitungsgeschwindigkeit oder die Mitverwendung von Harnstoff erforderlich sind.

Diese Aufgabe wird erfindungsgemäss dadurch gelöst, dass N-Monoalkyl-acetoacetamide und Chlor im Molverhältnis 1:0,8 bis 0,95 eingesetzt werden, als Reaktionsmedium ausschliesslich Wasser verwendet und die Umsetzung in flüssiger Phase bei Temperaturen im Bereich von −1°C bis −25°C in Gegenwart eines anorganischen Salzes, das dem Ausgangsgemisch in der zur Gefrierpunktserniedrigung auf die Reaktionstemperatur erforderlichen Menge zugegeben wird, durchgeführt wird, wobei die Mitverwendung von Harnstoff ausgeschlossen ist.

Bei dem erfindungsgemässen Verfahren können als Ausgangsprodukte die N-Monoalkylacetoacetamide entweder in reiner Form oder in Form konzentrierter wässriger Lösungen eingesetzt werden.

Beispiele für N-Monoalkylacetoacetamide sind N-Methyl, N-Ethyl, N-Isopropyl- und N-n-Propylacetoacetamide, wobei N-Methyl- und N-Ethylacetoamid bevorzugt sind.

Die Reaktionsteilnehmer, das sind N-Monoalkylacetoacetamide und Chlor, werden definitionsgemäss im Molverhältnis von 1:0,8 bis 0,95, vorzugsweise bis zu 0,90, eingesetzt. Ein Chlorüberschuss ist zu vermeiden, da hierdurch bereits die Selektivität störend beeinflusst wird.

Das erfindungsgemässe Verfahren kann bei Temperaturen im Bereich von −1°C bis −25°C diskontinuierlich oder kontinuierlich durchgeführt werden, wobei sich Temperaturen im Bereich von −18°C bis −23°C besonders bewährt haben.

Die entscheidende Massnahme bei der Durchführung des erfindungsgemässen Verfahrens besteht darin, dass als Reaktionsmedium ausschliesslich Wasser verwendet wird. Um sicher zu stellen, dass die Umsetzung auch im tieferen Temperaturbereich in flüssiger Phase erfolgt, wird dem Ausgangsgemisch die zur Gefrierpunktserniedrigung auf die Reaktionstemperatur erforderliche Menge eines anorganischen Salzes zugegeben, wobei sich die Chloride von Natrium, Calcium und/oder Magnesium besonders bewährt haben. Es können jedoch auch andere anorganische Salze, die sich gegenüber den Reaktionspartnern inert verhalten, wie $KH_2PO_4$ oder $K_2HPO_4$ verwendet werden. Zusätzlich kann durch mechanische Bewegung für eine gute Durchmischung der Reaktionspartner gesorgt werden.

Die zur Durchführung des erfindungsgemässen Verfahrens erforderliche Wassermenge kann in weitem Bereich variiert werden in Abhängigkeit von der Löslichkeit der Reaktionspartner und der vorhandenen reaktionsinerten anorganischen Salzzusätze. Je Gewichtsteil eingesetztes Acetoacetamid sind jedoch mindestens 0,5 Gewichtsteile Wasser notwendig. Es können jedoch auch bis zu 15 Gewichtsteile Wasser je Gewichtsteil Acetoacetamid eingesetzt werden. Für die obere Grenze sind in erster Linie verfahrenstechnische Gründe massgebend, da bekanntlich zur Erzielung einer guten Raum/Zeitausbeute ein geringes Reaktionsvolumen vorteilhaft ist. Vorzugsweise werden je Gewichtsteil Acetoacetamid 0,7 bis 7 Gewichtsteile Wasser verwendet.

Nach beendeter Chlorierung wird das Reaktionsgemisch in bekannter Weise aufgearbeitet,

das heisst, die Reaktionsprodukte werden aus der wässrigen Phase durch Extraktion mit einem mit Wasser nicht mischbaren organischen Lösungsmittel, wie Benzol, Toluol oder Chloroform abgetrennt. Eine Neutralisation der organischen Phase ist hierbei nicht erforderlich, sondern diese kann ohne zusätzliche Massnahmen weiter verarbeitet werden, das heisst, nach Abdestillieren des Extraktionsmittels und des bei der Chlorierung gebildeten Chlorwasserstoffs verbleibt als Rückstand das gewünschte alpha-Chloracetoacetamid.

Eine Neutralisation der wässrigen Phase ist hingegen nur dann erforderlich, wenn das Verfahren kontinuierlich durchgeführt wird und die wässrige Phase erneut als Reaktionsmedium für die Chlorierung eingesetzt, das heisst, im Kreislauf geführt wird, da hierin gelöster Chlorwasserstoff die Selektivität bei der erneuten Chlorierung stört. In diesem Falle ist es vorteilhaft, die Neutralisierung mit wässriger Natronlauge vorzunehmen, da hierdurch die für die Gefrierpunktserniedrigung des Reaktionsmediums benötigte Natriumchloridmenge gebildet wird, was eine weitere Vereinfachung der Verfahrensführung ermöglicht.

Nach dem erfindungsgemässen Verfahren können alpha-Chlor-Monoalkylacetoacetamide mit einer Selektivität von bis zu 99,5% bei einem Umsatz von über 95,5% und in einer Ausbeute von bis zu 95,5% der Theorie, bezogen auf umgesetztes Mono-N-alkylacetoacetamid hergestellt werden, was nach den bisher bekannten Verfahren nicht möglich war. Dieses Ergebnis muss insbesondere im Hinblick auf die Tatsache als überraschend bewertet werden, dass bisher davon ausgegangen wurde, dass die Steigerung der Wassermenge in einem Alkanol enthaltenden Reaktionsmedium eine Verminderung der Selektivität und des Umsatzes zur Folge hat.

Beispiel 1

Die bei der Chlorierung verwendete Apparatur bestand aus einem emaillierten Reaktionsrührwerk mit Einleitungsrohr, Kühlmantel und Bodenauslauf, der mit einem Schauglas zur Schichtentrennung verbunden war.

Das Reaktionsrührwerk wurde in der angegebenen Reihenfolge mit

773 Tl. Wasser,

227 Gew.-Tl. NaCl, techn. rein, und

115,7 Gew.-Tl. N-Methyl-acetoacetamid (MMAA), 99,5%-ig beschickt.

In diesem Gemisch wurden unter Rühren bei −19°C bis −22°C im Verlaufe von 1 Stunde insgesamt 56,7 Gew.-Tl. gasförmiges Chlor tauchend eingeleitet.

Anschliessend wurde das erhaltene Reaktionsgemisch bei Raumtemperatur erschöpfend mit Benzol extrahiert, die Benzol-Phase der destillativen Aufarbeitung zugeführt und die wässrige Phase mit 50%-iger Natronlauge bei Raumtemperatur neutralisiert. Bei der destillativen Aufarbeitung der Benzol-Phase wurde das Benzol durch einfaches Abtreiben im Vakuum (ca. 560 mbar, erst gegen Ende des Vorgangs wurde das Vakuum auf 6,5 mbar erhöht) praktisch vollständig entfernt, wobei eine Sumpftemperatur von 85°C nicht überschritten wurde. Als Rückstand wurde dabei eine bei 78°C erstarrende Schmelze erhalten, mit folgender Zusammensetzung:

97,4 Gew.% N-Methyl-α-chloracetoacetamid (MMCAA)

2,0 Gew.% N-Methylacetoacetamid (MMAA)

0,6 Gew.% N-Methyl-α-α-dichloracetoacetamid (MMDCAA).

Die neutrale, NaCl-haltige, wässrige Phase mit einem Rest-MMAA-Gehalt von 21,6 Gew.-Tl. wurde erneut in das Reaktionsrührwerk eingelegt, mit 50 Tl. Wasser und 94,0 Gew.-Tl. 99,5%-igem N-Methylacetoacetamid versetzt und wie oben beschrieben chloriert und aufgearbeitet.

Insgesamt wurde die wässrige Phase dabei 5 mal der Chlorierung zugeführt. Es wurden insgesamt 492 Gew.-Tl. N-Methylacetoacetamid (MMAA) 99,5%-ig, sowie 283,5 Gew.-Tl. Chlor eingesetzt und insgesamt 588,6 Gew.-Tl. 97,4%-iges N-Methyl-α-chloracetoacetamid (MMCAA) erhalten, das sind 96,8% d.Th. bezogen auf umgesetztes MMAA.

Im MMCAA sind noch 11,8 Gew.-Tl. MMAA enthalten. Der MMAA-Umsatz betrug 95,6% und die Selektivität 99,5%. Praktisch gleiche Ergebnisse wurden erhalten, wenn anstelle von NaCl 203 Gew.-Tl. $CaCl_2$ oder 163 Gew.-Tl. $MgCl_2$ eingesetzt wurden.

Beispiel 2 mit 3

Das Verfahren gemäss Beispiel 1 wurde wiederholt, jedoch unter Abänderung des Molverhältnisses Chlor:MMAA. Die Ergebnisse sind in der folgenden Tabelle I zusammengestellt:

Tabelle 1

| Beisp. Nr. | Molverhältn. Chlor:MMAA | Zusammensetzung des Produktes in MMCAA | MMAA | MMDCAA Gew.-% |
|---|---|---|---|---|
| 1 | 0,8:1 | 97,4 | 2,0 | 0,6 |
| 2 | 0,9:1 | 96,5 | 2,5 | 0,9 |
| 3 | 0,95:1 | 95,5 | 2,0 | 1,5 |

Beispiel 4

Das Verfahren gemäss Beispiel 1 wurde wiederholt, jedoch mit der Abänderung, dass anstelle von 99,5%-igen N-Methylacetoacetamid eine wässrige 68,7%-ige Lösung eingesetzt wurde, die durch Umsetzung von Diketen mit wässrigem 40%-igem Methylamin hergestellt worden war.

Insgesamt wurden 712 Gew.-Tl. einer 68,7%-igen N-Methylacetoacetamidlösung (MMAA) eingesetzt und 580,5 Gew.-Tl. N-Methyl-α-chloracetoacetamid (MMCAA) von der gleichen Zusammensetzung, wie in Beispiel 1 beschrieben, erhalten. Das sind 95,5% d.Th. bezogen auf umgesetztes MMAA.

Beispiel 5

Das Verfahren gemäss Beispiel 1 wurde wiederholt, jedoch mit der Abänderung, dass nach beendeter Chlorierung das Reaktionsgemisch abgekühlt und die Suspension filtriert, der dabei anfallende Feststoff mit Benzol extrahiert und der Extrakt wie in Beispiel 1 angegeben weiterbehandelt wurde. Dabei wurden als Rückstand 309,4 Gew.-Tl. einer bei 80°C erstarrenden Schmelze erhalten, die reines 99,5%-iges N-Methyl-2-chloracetoacetamid (MMCAA) darstellte, das sind 52% der Theorie bezogen auf umgesetztes MMAA. Die verbleibende Mutterlauge wurde wie in Beispiel 1 beschrieben, aufgearbeitet und daraus weitere 260 Gew.-Tl. MMCAA 100%-ig gerechnet, erhalten.

**Patentansprüche**

1. Verfahren zur Herstellung von alpha-Chlor-N-monoalkylacetoacetamiden der allgemeinen Formel

$CH_3COCH(Cl)CONR_1H$

worin $R_1$ Alkylreste mit 1 bis 3-C-Atomen bedeutet, durch Umsetzung von N-Monoalkyl-acetoacetamiden der allgemeinen Formel

$CH_3COCH_2CONR_1H$

worin $R_1$ die angegebene Bedeutung hat, mit Chlor in Gegenwart von Wasser enthaltenden Lösungsmitteln bei Temperaturen unter 0°C, dadurch gekennzeichnet, dass N-Monoalkyl-acetoacetamide und Chlor im Molverhältnis von 1:0,8 bis 0,95 eingesetzt werden, als Reaktionsmedium ausschliesslich Wasser verwendet und die Umsetzung in flüssiger Phase bei Temperaturen im Bereich von −1°C bis −25°C in Gegenwart eines anorganischen Salzes, das dem Ausgangsgemisch in der zur Gefrierpunktserniedrigung auf die Reaktionstemperatur erforderlichen Menge zugegeben wird, durchgeführt wird, wobei die Mitverwendung von Harnstoff ausgeschlossen ist.

2. Verfahren nach Anspruch 1, dadurch gekennzeichnet, dass N-Monoalkyl-acetoacetamide und Chlor im Molverhältnis 1:0,8 bis 0,9 eingesetzt werden.

3. Verfahren nach Anspruch 1, dadurch gekennzeichnet, dass als anorganische Salze Chloride von Natrium, Calcium und/oder Magnesium verwendet werden.

4. Verfahren nach Anspruch 1, dadurch gekennzeichnet, dass je Gewichtsteil Acetoacetamid 0,7 bis 7 Gewichtsteile Wasser eingesetzt werden.

**Claims**

1. Process for the manufacture of alpha-chlor-N-monoalkylacetoacetamides of the general formula

$CH_3COCH(Cl)CONR_1H$

in which $R_1$ represents alkyl radicals having from 1 to 3 carbon atoms, by reacting N-monoalkylacetoacetamides of the general formula

$CH_3COCH_2CONR_1H$

in which $R_1$ has the meaning given above, with chlorine in the presence of water-containing solvents at temperatures below 0°C, characterized in that N-monoalkyl-acetoacetamides and chlorine are used in the molar ratio of 1:0.8 to 0.95, that exclusively water is used as reaction medium and the reaction is carried out in the liquid phase at temperatures in the range of from −1°C to −25°C in the presence of an inorganic salt, that is added to the starting mixture in the amount required to reduce the freezing point to the reaction temperature, the concomitant use of urea being excluded.

2. Process according to claim 1, characterized in that N-monoalkyl-acetoacetamides and chlorine are used in the molar ratio of 1:0.8 to 0.9.

3. Process according to claim 1, characterized in that chlorides of sodium, clacium and/or magnesium are used as inorganic salts.

4. Process according to claim 1, charcterized in that 0.7 to 7 parts by weight of water are used per part by weight of acetoacetamide.

**Revendications**

1. Procédé de préparation d'α-chloro-N-monoalkyl-acétoacétamides répondant à la formule générale

$CH_3COCH(Cl)CONR_1H$

dans laquelle $R_1$ désigne un radical alkyle en $C_1$ à $C_3$, par réaction de N-monoalkyl-acétoacétamides répondant à la formule générale

$CH_3COCH_2CONR_1H$

dans laquelle $R_1$ a la signification indiquée, avec du chlore en présence de solvants contenant de

l'eau, à une température inférieure à 0°C, procédé caractérisé en ce qu'on met en jeu des N-monoalkyl-acétoacétamides et du chlore dans un rapport molaire de 1:0,8 à 0,95, en ce qu'on utilise exclusivement de l'eau comme milieu réactionnel, et en ce qu'un effectue la réaction en phase liquide, à des températures comprises entre −1°C et −25°C, et en présence d'un sel minéral, lequel est ajouté au mélange de départ en la quantité nécessaire pour abaisser le point de congélation à la température réactionnelle, l'emploi simultané d'urée étant exclu.

2. Procédé selon la revendication 1, caractérisé en ce qu'on met en jeu des N-monoalkyl-acétoacétamides et du chlore dans un rapport molaire de 1:0,8 à 0,9.

3. Procédé suivant la revendication 1, caractérisé en ce qu'on utilise comme sels minéraux des chlorures de sodium, de calcium et/ou de magnésium.

4. Procédé suivant la revendication 1, caractérisé en ce qu'on met en jeu de 0,7 à 7 parties en poids d'eau par partie en poids d'acétoacétamide.